(19) Europäisches Patentamt
European Patent Office
Office européen des brevets

(11) Veröffentlichungsnummer: **0 031 962 B1**

(12) **EUROPÄISCHE PATENTSCHRIFT**

(45) Veröffentlichungstag der Patentschrift: **09.02.83**

(51) Int. Cl.³: **C 07 F 7/18,** A 01 N 55/00

(21) Anmeldenummer: **80108221.5**

(22) Anmeldetag: **27.12.80**

(54) **Fungizide Azolyl-silyl-glykolether, ihre Herstellung, ihre Verwendung zur Bekämpfung von Fungi und Mittel dafür.**

(30) Priorität: **04.01.80 DE 3000140**

(43) Veröffentlichungstag der Anmeldung: **15.07.81 Patentblatt 81/28**

(45) Bekanntmachung des Hinweises auf die Patenterteilung: **09.02.83 Patentblatt 83/6**

(84) Benannte Vertragsstaaten: **AT BE CH DE FR GB IT LI NL SE**

(56) Entgegenhaltungen: **DE-A-2 838 847**

(73) Patentinhaber: **BASF Aktiengesellschaft, Carl-Bosch-Strasse 38, D-6700 Ludwigshafen (DE)**

(72) Erfinder: **Acker, Rolf-Dieter, Dr., Tuchbleice 8, 6906 Leimen (DE)**
Erfinder: **Buschmann, Ernst, Dr., Georg-Ludwig-Krebs-Strasse 10, D-6700 Ludwigshafen (DE)**
Erfinder: **Thym, Sabine, Dr., Hasenhain 20, D-6901 Dossenheim (DE)**
Erfinder: **Zeeh, Bernd, Dr., Thorwaldsenstrasse 5, D-6700 Ludwigshafen (DE)**
Erfinder: **Pommer, Ernst-Heinrich, Dr., Berliner Platz 7, D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Fungizide Azolyl-silyl-glykolether, ihre Herstellung, ihre Verwendung zur Bekämpfung von Fungi und Mittel dafür

Die vorliegende Erfindung betrifft neue Azolyl-silyl-glykolether, Verfahren zu deren Herstellung, deren Verwendung als Fungizide, fungizide Mittel, die diese Wirkstoffe enthalten, Verfahren zur Herstellung solcher fungiziden Mischungen sowie Verfahren zur Bekämpfung von Schadpilzen mit diesen Fungiziden oder mit fungiziden Mischungen, die diese Verbindungen enthalten.

Es ist bereits eine größere Anzahl biologisch aktiver Silicium-haltiger Verbindungen bekannt. Deren Anwendung beschränkt sich bisher im wesentlichen auf das Gebiet der Pharmakologie. (Journ. of Pharmaceutical Sciences, 60, 1113–1127 (1971), Deutsche Apotheker-Zeitung, 118, 1743–1747, (1978)). Über die Verwendbarkeit Si-organischer Substanzen im Pflanzenschutz wurde nur wenig publiziert (US-A-4 150 122, DE-A-2 208 329). Über Fungizide aus dieser Substanzklasse ist bislang nichts bekannt.

Es wurde nun gefunden, daß Azolyl-silyl-glykolether der Formel I

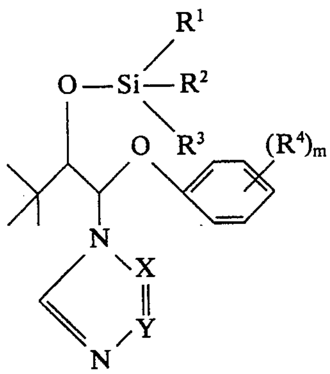

(I)

worin

$R^1$, $R^2$ und $R^3$ einen $C_{1-4}$-Alkylrest oder Phenyl,
$R^4$ einen $C_{1-4}$-Alkylrest, Halogen oder Phenyl,
m die Zahl 0, 1, 2 oder 3 und
X und Y CH oder N

bedeuten, sowie deren mit Pflanzen verträglichen Salze eine sehr gute fungizide Wirksamkeit besitzen.

$R^1$, $R^2$ und $R^3$, die voneinander verschieden sein können, bedeuten vorzugsweise Methyl, Ethyl oder n-Butyl.

$R^4$ ist vorzugsweise Chlor in der 2- und/oder 4-Stellung des Phenylringes, während m insbesondere 1 oder 2 bedeutet.

X und Y können gleich oder verschieden sein. Vorzugsweise sind X ein Stickstoffatom und Y eine CH-Gruppe oder X und Y CH-Gruppen.

Die neuen Azolyl-silyl-glykolether der Formel I lassen sich herstellen, indem man einen Azolylglykolether der Formel II

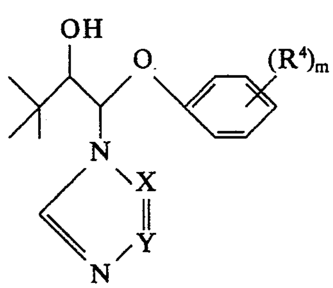

(II)

worin $R^4$, m, X und Y die angegebene Bedeutung haben,

a) mit einem Trialkylsilylamid der Formel III

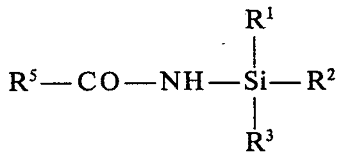

(III)

in der $R^1$, $R^2$ und $R^3$ die angegebene Bedeutung haben und $R^5$ Alkyl oder Aryl ist, oder

b) in Gegenwart eines säurebindenden Mittels mit einem Trialkylchlorsilan der Formel IV

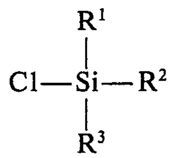

(IV)

in der $R^1$, $R^2$ und $R^3$ die angegebene Bedeutung besitzen,

umsetzt und die so erhaltenen Verbindungen gegebenenfalls in ihre mit Pflanzen verträglichen Salze überführt.

Die Umsetzung der Verbindungen II mit den Trialkylsilylamiden III erfolgt zweckmäßigerweise bei einer Reaktionstemperatur von 40 bis 140°C in einem organischen Lösungsmittel wie Tetrahydrofuran, Dioxan, Benzol, Toluol oder Xylol.

Geeignete Silylamide der Formel III sind beispielsweise: N-Trimethylsilylacetamid, N-Triethylsilylacetamid, N-(butyl-dimethyl-silyl)-acetamid und N-Trimethylsilylbenzamid.

Die Reaktion von II mit den Trialkylchlorsilanen IV gelingt gut bei −5 bis +30°C, indem man beispielsweise zu einer Lösung von II und des Säurefängers in einem geeigneten Lösungsmittel wie THF, Dioxan, $CHCl_3$, $CH_2Cl_2$ das Trialkylchlorsilan zutropft. Geeignete Säurefänger sind beispielsweise: Diisopropylethylamin, Triethylamin, Pyridin. Vorteilhaft ist dabei die Zugabe katalytischer Mengen 4-Dimethylaminopyridin.

Geeignete Trialkylchlorsilane IV sind beispielsweise: Trimethylchlorsilan, Triethylchlorsilan, n-Butyl-dimethylchlorsilan und tert.-Butyl-dimethylchlorsilan.

Die neuen Verbindungen fallen in der Regel als Diastereomerengemische an, die gegebenenfalls nach bekannten Verfahren getrennt werden können.

Obwohl von Silylethern allgemein bekannt ist, daß sie unter neutralen Bedingungen leicht hydrolysieren, sind die erfindungsgemäßen Azolylsilylglykolether I überraschend hydrolysebeständig. Beispielsweise konnte in einer wäßrigen methanolischen Lösung des Wirkstoffs 1 nach drei Wochen spektroskopisch und chromatographisch kein Hydrolyseprodukt entdeckt werden.

Die folgenden Beispiele erläutern die Herstellung der erfindungsgemäßen Verbindungen I.

## Beispiel 1

1-p-Chlorphenoxy-3,3-dimethyl-2-trimethylsiloxy-1-(1,2,4-triazol-1-yl)-butan

Eine Lösung von 10 g 1-p-Chlorphenoxy-3,3-dimethyl-1-(1,2,4-triazol-1-yl)-butan-2-ol und 10 g N-Trimethylsilylacetamid in 100 ml wasserfreiem Toluol wird 6 h auf 100°C erwärmt. Zeigt die Dünnschichtchromatographie nach dieser Zeit, daß noch Ausgangsverbindung vorhanden ist, werden nochmals 5 g N-Trimethylsilylacetamid zugegeben und weitere 3 h auf 100°C erwärmt. Man läßt abkühlen, gibt weitere 400 ml Toluol zu, wäscht dreimal mit 250 ml $H_2O$, trocknet über $Na_2SO_4$ und engt ein. Das zurückbleibende Produkt zeigt im NMR ($\delta$-Werte, $CDCl_3$) zwei Diastereomere 1a und 1b von 1-p-Chlor-phenoxy-3,3-dimethyl-2-trimethylsiloxy-1-(1,2,4-triazol-1-yl)-butan.

1a:

$\delta$=0,01 (s, 9H) $SiC(CH_3)_3$; 0,96 (s, 9H) $C(CH_3)_3$; 3,85 (d, J=5 Hz, 1 H), 1 h) CHO; 6,10 (d, J=5 Hz, 1 H) NCHO; 6,63−7,25 (4 H) Aromat; 7,92 (s, 1 H); 8,12 (s, 1 H) Triazol.

1b:

$\delta$=0,25 (s, 9H), 0,75 (s, 9H); 3,92 (d, J=3 Hz, 1 H); 6,22 (d, J=3 Hz, 1 H); 6,63−7,25 (4 H); 7,92 (s, 1 H), 8,37 (s, 1 H).

## Beispiel 2

### 1-p-Bromphenoxy-3,3-dimethyl-2-trimethyl-siloxy-1-(1,2,4-triazol-1-yl)-butan

Zu einer Lösung von 3,4 g 1-p-Bromphenoxy-3,3-dimethyl-1-(1,2,4-triazol-1-yl)-butan-2-ol und 1,5 g Triethylamin in 40 ml Tetrahydrofuran wird bei 0° C eine Lösung von 8,5 g Trimethylchlorsilan in 20 ml THF zugetropft. Man rührt über Nacht bei Raumtemperatur, dampft ein, nimmt mit $CHCl_3$ auf, wäscht mehrfach mit Wasser und trocknet über $Na_2SO_4$. Nach dem Eindampfen wird das Rohprodukt chromatographiert ($SiO_2$, $CHCl_3$). Man erhält 2 g 1-p-Bromphenoxy-3,3-dimethyl-2-trimethylsiloxy-1-(1,2,4-triazol-1-yl)-butan als farbloses Harz.

Das NMR-Spektrum zeigt zwei Diastereomere.

2a:

$\delta = 0{,}05$ (s, 9H), $Si(CH_3)_3$; 1,05 (s, 9 H) $C(CH_3)_3$; 8,0 (s, 1 H), 8,50 (s, 1H) (Triazol).

2b:

$\delta = 0{,}35$ (s, 9 H) $Si(CH_3)_3$; 0,82 (s, 9 H) $C(CH_3)_3$; 8,07 (s, 1 H); 8,28 (s, 1 H) (Triazol).

## Beispiel 3

### 1-p-Phenylphenoxy-3,3-dimethyl-2-triethylsiloxy-1-(1,2,4-triazol-1-yl)-butan

5,5 g 1-p-Phenylphenoxy-3,3-dimethyl-1-(1,2,4-triazol-1-yl)-butan-2-ol werden in 25 ml THF gelöst. 2,9 g Triethylchlorsilan und 0,2 g 4-Dimethylaminopyridin werden unter Stickstoffatmosphäre zugefügt. Man erwärmt die Mischung auf 45° C und gibt portionsweise 3,3 g Triethylamin hinzu. Nach 14 h Rühren bei 40–50° C wird auf Raumtemperatur abgekühlt, vom Ungelösten filtriert und eingeengt. Die weitere Reinigung des Produktes erfolgt entweder durch Zugabe von Petrolether und erneutem Filtrieren und Einengen oder über eine Gelfiltration ($SiO_2$, $CHCl_3$). Man erhält 3 g 1-p-Phenylphenoxy-3,3-dimethyl-2-triethylsiloxy-1-(1,2,4-triazol-1-yl)-butan.

Analog Beispiel 1—3 wurden hergestellt:

| Beispiel Nr. | $R^1$ | $R^2$ | $R^3$ | $(R^4)_m$ | X | Y | δ-Werte NMR ($CDCl_3$) $SiR^1R^2R^3$ | $C(CH_3)_3$ |
|---|---|---|---|---|---|---|---|---|
| 4 | $CH_3$ | $CH_3$ | $CH_3$ | 2,4-$Cl_2$ | N | CH | 0,01; 0,25 | 0,75; 0,87 |
| 5 | $CH_3$ | $CH_3$ | $CH_3$ | 4-$C_6H_5$ | N | CH | 0,0; 0,28 | 0,79; 0,98 |
| 6 | $CH_3$ | $CH_3$ | $CH_3$ | 4-Cl | CH | CH | 0,03; 0,13 | 0,71; 0,73 |
| 7 | $CH_3$ | $CH_3$ | $CH_3$ | 2,4-$Cl_2$ | CH | CH | 0,21 | 0,8 |
| 8 | $CH_3$ | $CH_3$ | $CH_3$ | 2-Cl | CH | N | 0,1 | 0,87 |
| 9 | $C_2H_5$ | $C_2H_5$ | $C_2H_5$ | 4-Cl | N | CH | 0,3—1,1 | 0,99 |
| 10 | $C_2H_5$ | $C_2H_5$ | $C_2H_5$ | 4-Br | N | CH | 0,4—1,2 | 0,89; 0,95 |
| 11 | $C_2H_5$ | $C_2H_5$ | $C_2H_5$ | 2,4-$Cl_2$ | N | CH | 0,3—1,2 | 0,92; 0,95 |
| 12 | $CH_3$ | $CH_3$ | $nC_4H_9$ | 4-Cl | N | CH | 0,01; 0,3<br>0,0—1,4 | 0,77; 1,0 |
| 13 | $CH_3$ | $CH_3$ | $nC_4H_9$ | 4-Br | N | CH | 0,01—0,3<br>0,0—1,5 | 0,78; 1,0 |
| 14 | $CH_3$ | $CH_3$ | $nC_4H_9$ | 4-$C_6H_5$ | N | CH | 0,0; 0,3—1,5 | 1,0 |
| 15 | $CH_3$ | $CH_3$ | $nC_4H_9$ | 2,4-$Cl_2$ | N | CH | 0,01; 0,33<br>0,0—1,5 | 0,79; 0,90 |
| 16 | $CH_3$ | $CH_3$ | $CH_3$ | 4-$CH_3$ | N | CH | | |
| 17 | $CH_3$ | $CH_3$ | $C_6H_5$ | 4-$C_6H_5$ | N | CH | | |
| 18 | $CH_3$ | $CH_3$ | t-$C_4H_9$ | 4-Cl | N | CH | 0,10; 0,90 | 1,05 |
| 19 | $CH_3$ | $CH_3$ | $C_6H_5$ | 4-Cl | N | CH | | |
| 20 | $CH_3$ | $CH_3$ | $C_6H_5$ | 4-$C_6H_5$ | N | CH | 0,20; 0,25<br>7,0—7,4 | 0,90 |
| 21 | $CH_3$ | $CH_3$ | $C_6H_5$ | 2,4-$Cl_2$ | N | CH | | |
| 22 | $CH_3$ | $CH_3$ | $C_6H_5$ | 4-Br | N | CH | | |

Die erfindungsgemäßen Verbindungen und ihre Salze zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie in Gemüsen wie Gurken, Bohnen oder Kürbisgewächsen.

Die neuen Verbindungen sind insbesondere geeignet zur Bekämpfung folgender Pflanzenkrankheiten:

Erysiphe graminis (echter Mehltau) in Getreide,
Erysiphe cichoriacearum (echter Mehltau) an Kürbisgewächsen,
Podosphaera leucotricha an Äpfeln,
Uncinula necator an Reben,
Erysiphe polygoni an Bohnen,
Sphaerotheca pannosa an Rosen,

Puccinia-Arten an Getreide,
Rhizoctonia solani an Baumwolle sowie Helminthosporiumarten an Getreide,
Ustilago-Arten an Getreide und Zuckerrohr,
Rhynchosporium secale an Getreide,
Venturia inaequalis (Apfelschorf),
Hemileia vastatrix an Kaffee,
Mycosphaerella musicola an Bananen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die erfindungsgemäßen Substanzen lassen sich sehr gut formulieren und zeichnen sich durch eine sehr hohe Stabilität aus. Sie können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin, Dimethylformamid) und Wasser; Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel wie nichtionogene und anionische Emulgatoren (z. B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff. Die Aufwandmengen liegen je nach Art des gewünschten Effektes zwischen 0,03 und 3 kg Wirkstoff oder mehr je ha.

Die Teilchengröße der Wirkstoffe in den Formulierungen kann die fungizide Wirksamkeit beeinflussen. Bei einer Reihe von Verbindungen nimmt die fungizide Wirksamkeit mit abnehmender Teilchengröße zu.

Die neuen Verbindungen können auch in Materialschutz u. a. zur Bekämpfung holzzerstörender Pilze wie Coniophora puteanea und Polystictus versicolor eingesetzt werden; lösemittelhaltige Holzschutzmittel enthalten 0,05 bis 5 Gew.-% Wirkstoff, bezogen auf das Gesamtgewicht der Formulierung. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung des Beispiels 1 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.

III. 20 Gewichtsteile der Verbindung des Beispiels 3 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

IV. 20 Gewichtsteile der Verbindung des Beispiels 4 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

V. 20 Gewichtsteile der Verbindung des Beispiels 7 werden den mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfitablauge und 60 Gewichtsteilen pulverförmigem Kieselsäuregel

gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

VI. 3 Gewichtsteile der Verbindung des Beispiels 2 werden mit 97 Gewichtsteilen feinteiligem Kaolin innig vermischt. Man erhält auf diese Weise ein Stäubemittel, das 3 Gew.-% des Wirkstoffs enthält.

VII. 30 Gewichtsteile der Verbindung des Beispiels 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung des Beispiels 3 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.-% Wirkstoff enthält.

IX. 20 Teile der Verbindung des Beispiels 4 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z. B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
- Ferridimethyldithiocarbamat,
- Zink-dimethyldithiocarbamat,
- Manganethylenbisdithiocarbamat,
- Mangan-Zink-ethylendiamin-bis-dithiocarbamat und Zink-ethylenbisdithiocarbamat,
- Tetramethylthiuramdisulfide,
- Ammoniak-Komplex von Zink-[(N,N'-ethylen-bis-(dithiocarbamat)] und N,N'-Polyethylen-bis-(thiocarbamoyl)-disulfid,
- Zink-[N,N'-propylen-1,2-bis-(dithiocarbamat)],
- Ammoniak-Komplex von Zink-(N,N'-propylen-bis-dithiocarbamat) und N,N'-Poly-1,2-propylen-bis-(thiocarbamoyl)-disulfid;

Nitroderivate, wie
- 2,4-Dinitro-6-(1-methylheptyl)-phenylcrotonat,
- 2-sec-Butyl-4,6-dinitrophenyl-3,3-dimethylacrylat,
- 2-sec-Butyl-4,6-dinitrophenyl-isopropylcarbonat;

heterocyclische Substanzen, wie
- N-(1,1,2,2-Tetrachlorethylthio)-tetrahydrophthalimid,
- N-Trichlormethylthio-tetrahydrophthalimid,
- N-Trichlormethylthio-phthalimid,
- 2-Heptadecyl-2-imidazolin-acetat,
- 2,4-Dichlor-6-(o-chloranilino)-s-triazin,
- O,O-Diethyl-phthalimidophosphonothioate,
- 5-Amino-1-(bis-(dimethylamino)-phosphinyl)-3-phenyl-1,2,4-triazol,
- 5-Ethoxy-3-trichlormethyl-1,2,4-thiadiazol,
- 2,3-Dicyano-1,4-dithioanthrachinon,
- 2-Thio-1,3-dithio-(4,5-b)-chinoxalin,
- 1-(Butylcarbamoyl)-2-benzimidazol-carbaminsäuremethylester,
- 2-Methoxycarbonylamino-benzimidazol,
- 2-Rhodanmethylthio-benzthizol,
- 4-(2-Chlorphenylhydrazono)-3-methyl-5-isoxazolon,
- Pyridin-2-thio-1-oxid,
- 8-Hydroxychinolin bzw. dessen Kupfersalz,
- 2,3-Dihydro-5-carboxanilido-6-methyl-1,4-oxathiin-4,4-dioxid,
- 2,3-Dihydro-5-carboxanilido-6-methyl-1,4-oxathiin,
- 2-(Furyl-(2))-benzimidazol,
- N,N'-[Piperazin-1,4-diyl-bis-(2,2,2-trichlor-ethyliden)]-bis-(formamid),

2-(Thiazolyl-(4))-benzimidazol,
5-Butyl-2-dimethylamino-4-hydroxy-6-methyl-pyrimidin,
3-[Hydroxy-bis-(4-chloro-phenyl)methyl]-pyridin,
und weitere Substanzen wie
1,2-Bis-(3-ethoxycarbonyl-2-thioureido)-benzol,
1,2-Bis-(3-methoxycarbonyl-2-thioureido)-benzol,
Dodecylguanidinacetat,
3-(2-(3,5-dimethyl-2-oxocyclohexyl)-2-hydroxyethyl)-glutaramid,
Hexachlorbenzol,
N-Dichlorfluormethylthio-N',N'-dimethyl-N-phenyl-sulfamid,
2,5-Dimethyl-furan-3-carbonsäureanilid,
2,5-Dimethyl-furan-3-carbonsäure-cyclohexylamid,
2-Cyan-N-(ethylaminocarbonyl)-2-(methoxyimino)-acetamid,
2-Methyl-benzoesäure-anilid,
2-Jod-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze.
DL-Methyl-N-(2,6-dimethyl-phenyl)-N-furyl(2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-1')-1-(4'-chlorphenoxy)-3,3-dimethylbutan-2-on,
1-(1',2',4'-Triazolyl-1')-1-(4'-chlorphenoxy)-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolylharnstoff.

Die folgenden Beispiele A und B zeigen die biologische Wirkung der neuen Substanzen. Als Vergleichssubstanz diente das aus der DE-A-2 063 857 bekannte 1-[2'-(2",4"-Dichlorphenyl)-2'-(2"-propenyloxy)-ethyl]-1H-imidazol.

## Beispiel A

Blätter von in Töpfen gewachsenen Weizenkeimlingen der Sorte »Jubilar« werden mit wäßrigen Emulsionen aus 80% (Gew.-%) Wirkstoff und 20% Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22° C und 75 bis 80% relativer Luftfeuchtigkeit aufgestellt. Nach 10 Tagen wird das Ausmaß der Mehltauentwicklung ermittelt.

| Wirkstoff des Beispiels | Befall der Blätter nach Spritzung mit ...%iger Wirkstoffbrühe | | |
|---|---|---|---|
| | 0,025 | 0,006 | 0,0015 |
| 1 | 0 | 0 | 0 |
| 2 | 0 | 0 | 0 |
| 4 | 0 | 0 | 0 |
| 5 | 0 | 0 | 1 |
| 6 | 0 | 0 | 0 |
| 7 | 0 | 0 | 0 |
| 8 | 0 | 1 | 1 |
| Vergleichssubstanz | 2 | 4 | 5 |
| Kontrolle (unbehandelt) | | 5 | |

0 = kein Pilzbefall, abgestuft bis 5 = Totalbefall.

Beispiel B

Blätter von in Töpfen gewachsenen Weizensämlingen der Sorte »Caribo« werden mit Sporen des Braumrostes (Puccinia recondita) bestäubt. Danach werden die Töpfe für 24 Stunden bei 20 bis 22° C in eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschließend mit 0,012-, 0,006- und 0,0015%igen (Gew.-%) wäßrigen Spritzbrühen, die 80% Wirkstoff und 20% Ligninsulfonat in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70% relativer Luftfeuchte aufgestellt. Nach 8 Tagen wird das Ausmaß der Rostpilzentwicklung auf den Blättern ermittelt.

| Wirkstoff des Beispiels | Befall der Blätter nach Spritzung mit . . .%iger Wirkstoffbrühe | | |
|---|---|---|---|
| | 0,025 | 0,006 | 0,0015 |
| 1 | 0 | 0 | 3 |
| 2 | 0 | 1 | 2 |
| 4 | 0 | 2 | 4 |
| 5 | 0 | 0 | 0 |
| 6 | 0 | 2 | 2 |
| 7 | 0 | 2 | 2 |
| Vergleichssubstanz | 3 | 5 | 5 |
| Kontrolle (unbehandelt) | 5 | 5 | |

0 = kein Pilzbefall, abgestuft bis 5 = Totalbefall.

**Patentansprüche**

1. Azolyl-silyl-glykolether der Formel I

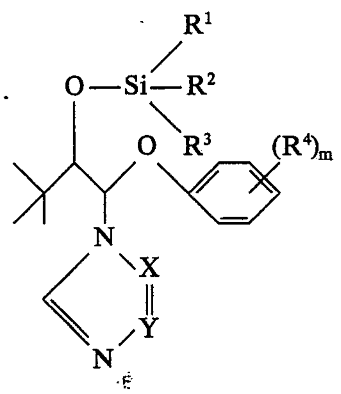

(I)

worin

$R^1$, $R^2$ und $R^3$ einen $C_{1-4}$-Alkylrest oder Phenyl,
$R^4$ einen $C_{1-4}$-Alkylrest, Halogen oder Phenyl,
m die Zahl 0, 1, 2 oder 3 und
X und Y CH oder N

bedeuten, sowie deren mit Pflanzen verträgliche Salze.

2. Verbindungen gemäß Anspruch 1, dadurch gekennzeichnet, daß X ein Stickstoffatom und Y eine CH-Gruppe sind.

3. Verbindungen gemäß Anspruch 1, dadurch gekennzeichnet, daß X und Y CH-Gruppen sind.

4. Azolylglykolsilylether gemäß Anspruch 1, ausgewählt aus der Gruppe bestehend aus

1-p-Chlorphenoxy-3,3-dimethyl-2-trimethylsiloxy-1-(1,2,4-triazol-1-yl)-butan,
1-(2,4-Dichlorphenoxy)-3,3-dimethyl-2-trimethylsiloxy-1-(1,2,4-triazol-1-yl)-butan,
3,3-Dimethyl-2-trimethylsiloxy-1-(4-phenylphenoxy)-1-(1,2,4-triazol-1-yl)-butan.

5. Verfahren zur Herstellung der Azolyl-silyl-glykolether der Formel I gemäß Anspruch 1, dadurch gekennzeichnet, daß man einen Azolylglykolether der Formel II

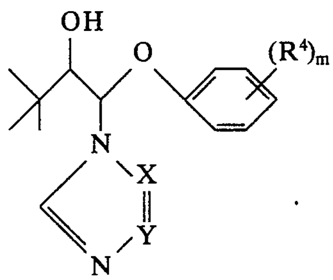

(II)

worin $R^4$, m, X und Y die angegebene Bedeutung haben,

a) mit einem Trialkylsilylamid der Formel III

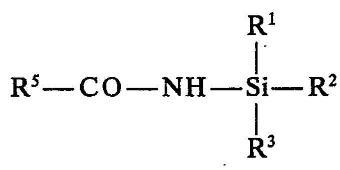

(III)

in der $R^1$, $R^2$ und $R^3$ die angegebene Bedeutung haben und $R^5$ Alkyl oder Aryl ist, oder

b) in Gegenwart eines säurebindenden Mittels mit einem Trialkylchlorsilan der Formel IV

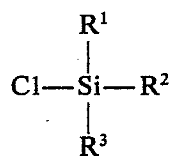

(IV)

in der $R^1$, $R^2$ und $R^3$ die angegebene Bedeutung besitzen,

umsetzt und die so erhaltenen Verbindungen gegebenenfalls in ihre mit Pflanzen verträglichen Salze überführt.

6. Fungizides Mittel, enthaltend mindestens eine Verbindung gemäß Anspruch 1.

7. Fungizides Mittel, enthaltend mindestens eine Verbindung gemäß Anspruch 1 und einen festen oder flüssigen Trägerstoff.

8. Verfahren zur Bekämpfung von Fungi, dadurch gekennzeichnet, daß man fungizide Mittel gemäß Anspruch 5 oder 6 auf diese einwirken läßt.

9. Verfahren zur vorbeugenden Bekämpfung von Fungi, dadurch gekennzeichnet, daß man mindestens eine Verbindung gemäß Anspruch 1 auf durch Pilzbefall bedrohte Flächen, Pflanzen oder Saatgüter einwirken läßt.

10. Verwendung von Verbindungen der Formel I gemäß Anspruch 1 bei der Bekämpfung von Fungi.

## Claims

1. An azolyl-silyl-glycol ether of the formula I

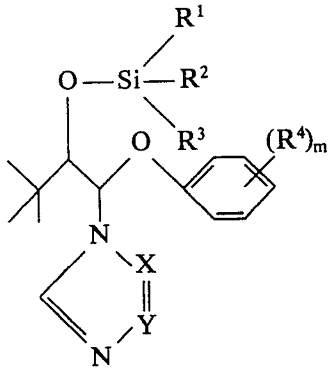

(I)

where

$R^1$, $R^2$ and $R^3$ denote $C_{1-4}$-alkyl or phenyl,
$R^4$ denotes $C_{1-4}$-alkyl, halogen or phenyl,
m denotes the integer 0, 1, 2 or 3, and
X and Y denote CH or N,

or a salt thereof which is tolerated by plants.

2. A compound as claimed in claim 1, wherein X is a nitrogen atom and Y is a CH group.

3. A compound as claimed in claim 1, wherein both X and Y denote CH groups.

4. An azolyl-silyl-glycol ether as claimed in claim 1, selected from the group consisting of

1-p-chlorophenoxy-3,3-dimethyl-2-trimethylsiloxy-1-(1,2,4-triazol-1-yl)-butane,
1-(2,4-dichlorophenoxy)-3,3-dimethyl-2-trimethylsiloxy-1-(1,2,4-triazol-1-yl)-butane and
3,3-dimethyl-2-trimethylsiloxy-1-(4-phenylphenoxy)-1-(1,2,4-triazol-1-yl)-butane.

5. A process for manufacturing an azolyl-silyl-glycol ether of the formula I as claimed in claim 1, wherein an azolylglycol ether of the formula II

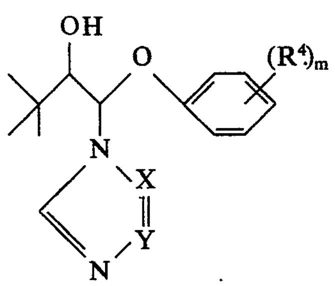

(II)

where $R^4$, m, X and Y have the meanings given, is reacted

a) with a trialkylsilyl amide of the formula III

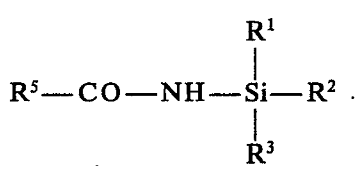

(III)

where $R^1$, $R^2$ und $R^3$ have the meanings given, and $R^5$ is alkyl or aryl, or

b) in the presence of an acid-binding agent – with a trialkylchlorosilane of the formula IV

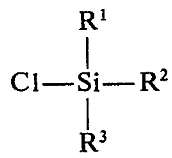

(IV)

where $R^1$, $R^2$ and $R^3$ have the meanings given,

and the compound thus obtained is converted, if desired, into a salt tolerated by plants.

6. A fungicidal agent containing at least one compound as claimed in claim 1.

7. A fungicidal agent containing at least one compound as claimed in claim 1 and a solid or liquid carrier.

8. A process for combating fungi, wherein a fungicidal agent as claimed in claim 5 or 6 is allowed to act thereon.

9. A process for the prophylactic control of fungi, wherein at least one compound as claimed in claim 1 is allowed to act on areas, plants or seed threatened by fungus attack.

10. The use of compounds of the formula I as claimed in claim 1 for combating fungi.

**Revendications**

1. Azolyl-silyl-glycolethers de formule I

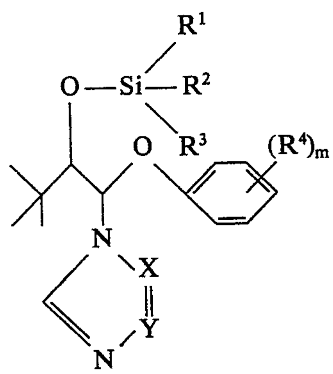

(I

où

$R^1$, $R^2$ et $R^3$ représentent un reste alkyle en $C_{1-4}$ ou phényle
$R^4$ un reste alkyle en $C_{1-4}$, halogène ou phényle
m le nombre 0, 1, 2 ou 3 et
X et Y, CH ou N

ainsi que leurs sels compatibles avec les plantes.

2. Composés selon la revendication 1, caractérisés par le fait que X est un atome d'azote et Y ui groupe CH.

3. Composés selon la revendication 1, caractérisés par le fait que X et Y sont des groupes CH.

4. Azolylglykolsilyléther selon la revendication 1, choisi dans le groupe constitué de:

1-p-chlorophénoxy-3,3-diméthyl-2-triméthylsiloxy-1-(1,2,4-triazol-1-yl)-butane,
1-(2,4-dichlorophénoxy)-3,3-diméthyl-2-triméthylsiloxy-1-(1,2,4-triazol-1-yl)-butane,
3,3-diméthyl-2-triméthylsiloxy-1-(4-phénylphénoxy)-1-(1,2,4-triazol-1-yl)-butane.

5. Procédé de préparation de azolyl-silylglycolethers de formule I selon la revendication 1 caractérisé par le fait qu'on fait réagir un azolylglycolether de formule II

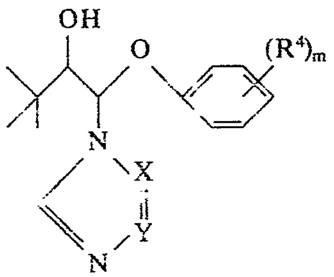

(II)

où $R^4$, m, X et Y ont les significations indiquées,

a) avec un trialkylsilylamide de formule III

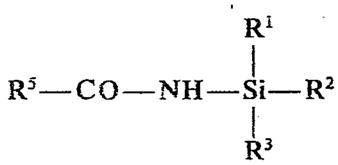

(III)

dans laquelle $R^1$, $R^2$ et $R^3$ ont les significations indiquées et $R^5$ est un alkyle ou aryle, ou

b) en présence d'un agent liant les acides, avec un trialkylchlorosilane de formule IV

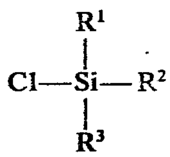

(IV)

dans laquelle $R^1$, $R^2$ et $R^3$ ont les significations indiquées,

et l'on transforme éventuellement les composés ainsi obtenus en leurs sels compatibles avec des plantes.

6. Fongicide, contenant au moins un composé selon la revendication 1.

7. Fongicide, contenant au moins un composé selon la revendication 1 et un support solide ou liquide.

8. Procédé de lutte contre les champignons, caractérisé par le fait qu'on fait agir sur ceux-ci un fongicide selon la revendication 5 ou 6.

9. Procédé de lutte préventive contre les champignons, caractérisé par le fait qu'on fait agir au moins un composé selon la revendication 1 sur des surfaces, plantes ou graines menacées par l'attaque de ces champignons.

10. Utilisation de composés de formule I selon la revendication 1 pour la lutte contre les champignons.